# EUROPEAN PATENT APPLICATION

(11) **EP 3 808 451 A1**
(43) Date of publication of application: **21.04.2021**
(21) Application number: 19203918.8
(22) Date of filing: 17.10.2019
(51) Int. Cl.: B01J 37/20, B01J 23/89, B01J 27/053, C07C 5/22

(54) **SULFATED METAL OXIDE CATALYST FOR ISOMERIZATION OF PARAFFIN HYDROCARBONS**

(71) Applicant: SABIC Global Technologies B.V., 4612 PX Bergen op Zoom (NL)
(72) Inventor: DHACHAPALLY, Naresh, 562125 Bengaluru, Karnataka (IN); SREEKANTH, Pavani, 562125 Bengaluru, Karnataka (IN); HASYAGAR, Umesh Krishna, 562125 Bengaluru, Karnataka (IN); BADGANDI, Srikant, 562125 Bengaluru, Karnataka (IN); NAIR, Vinod S, 562125 Bengaluru, Karnataka (IN)
(74) Representative: J A Kemp LLP

(57) **Abstract**

Disclosed are supported catalyst compositions for catalyzing isomerization of n-paraffin hydrocarbons, process of preparing the supported catalyst composition and process of producing isoparaffin hydrocarbons by isomerization of n-paraffin hydrocarbons using the supported catalyst composition. The supported catalyst composition contains, a noble metal and a sulfated support, the sulfated support contains an oxide or hydroxide of a group IV element and/or of aluminum, a group VIII metal oxide and a group V metal oxide or a group VI metal oxide or both, with weight ratio of the noble metal to the group VIII metal is 1:1 to 1:5.

## Description

### CROSS REFERENCE TO RELATED APPLICATIONS

None.

### FIELD OF INVENTION

The invention generally concerns supported catalyst compositions for isomerization of n-paraffin hydrocarbons. In particular, the invention concerns supported catalyst compositions containing a noble metal and a sulfated support. The sulfated support can include an oxide or hydroxide of a group IV element and/or of aluminum, a group VIII metal oxide, and a group V metal oxide and/or a group VI metal oxide.

### BACKGROUND OF THE INVENTION

The industrial importance of upgrading light hydrocarbons in refineries and gas processing plants has increased dramatically in recent years. Skeletal isomerization of n-paraffin hydrocarbons, such as n-butane has a specific interest in the field of petroleum refining industry with an aim to produce hydrocarbons of high octane number. Iso-butane obtained from n-butane isomerization is a valuable precursor for the production of alkylated gasoline and oxygenates used as octane number boosters, such as methyl tert-butyl ether (MTBE), ethyl tert-butyl ether (ETBE), and glycerol tert-butyl ether.

Industrial processes for production of isobutane include isomerization of n-butane over liquid and solid acid catalysts. Traditional industrial liquid acid catalysts, such as H₂SO₄, HF, and SbF₅/HSO₃F have undesired drawbacks, which attribute to their corrosivity and difficulty of recover and reuse. Solid acids are the more powerful catalysts for replacement of conventional harmful mineral acids in order to develop green processes. In the past century, a number of n-paraffin hydrocarbons isomerization technologies were commercialized based on solid acid catalysts. Such technologies include isomerization at high temperatures (360-440 °C) over fluorinated Pt/alumina catalysts, isomerization at medium temperatures (250-300 °C) over Pt/zeolite catalysts, and isomerization at low temperatures (120-190 °C) over Pt/Al₂O₃-Cl catalysts. The low temperature process are generally advantageous over higher temperature with increased selectivity and decreased operational cost. However, platinum supported chlorinated alumina catalysts have many disadvantages such as, continuous supply of chlorinated agents are used to maintain the catalyst activity and accordingly dry gas alkali treatment section is typically needed. Additionally, corrosion-proof materials are used for chlorine and wastes disposal, which often is environmentally damaging. Moreover the catalysts have highly sensitive to impurities (e.g., sulfur, nitrogen and water), accordingly upstream feed treatment is typically needed.

### SUMMARY OF THE INVENTION

A discovery has been made that provides a solution to at least some of the aforementioned problems associated with the isomerization of n-paraffin hydrocarbons. The solution is premised on performing isomerization of n-paraffin hydrocarbons using a supported catalyst composition that is structured such that the support material can include a sulfated support comprising an oxide or hydroxide of a group IV element and/or of aluminum, a group VIII metal oxide, a group V metal oxide or a group VI metal oxide or both, and a noble metal supported by the sulfated support. The noble metal can be deposited on the surface of the support or distributed throughout the support, or both. In certain instances, the weight ratio of the noble metal to the group VIII metal in the catalyst composition is 1:1, 1:2, 1:3, 1:4 to 1:5. As illustrated in non-limiting embodiments of the present invention, this supported catalyst composition can provide for a relatively high conversion, selectivity, and yield for isomerization C4-C7 n-paraffin hydrocarbons to C4-C7 iso-paraffin hydrocarbons. Further, and in certain aspects, the supported catalyst composition has limited to no chlorine content (e.g., less, than 0.5 wt%, preferably less than 0.1 wt, or more preferably no chlorine).

In some aspects, the catalyst composition can contain 0.1, 0.2, 0.3, 0.4, 0.5, 0.6, 0.7, 0.8, 0.9 wt. % to 1 wt. % of the noble metal. In some aspects, the composition can contain 0.5, 1.0, 1.5, 2.0, 2.5, 3.0, 3.5, 4.0, 4.5 wt. % to 5 wt. % of the group VIII metal. In some aspects, the composition can contain 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 wt. % to 2 wt. % of the group V metal. In some aspects, the composition can contain 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 wt. % to 2 wt. % of the group VI metal. In some aspects, the composition can contain 50, 55, 60, 65, 70, 75 wt. % to 80 wt. % of the group IV element. In some aspects, the composition can contain 50, 55, 60, 65, 70, 75 wt. % to 80 wt. % of aluminum, as a oxide or hydroxide. In some aspects, the composition can contain 1.0, 1.5, 2.0, 2.5, 3.0, 3.5, 4.0, 4.5 wt. % to 5.0 wt. % of sulfur, present as a sulfate (SO₄²⁻). In some aspects, weight ratio of the noble metal to the group VI metal in the catalyst composition can be between 1:0.5, 1:0.75, 1:1, 1:1.25 to 1:1.5. In some aspects, weight ratio of the noble metal to the group V metal in the catalyst composition can be 1:0.1, 1:0.2, 1:0.3, 1:0.4, 1:0.5, 1:0.6, 1:0.7, 1:0.8, 1:0.9, 1:1.0, 1:1.1 to 1:1.2. In some aspects, the group IV element can be zirconium. In some particular aspects, the oxide of the group IV element can be zirconia (ZrO₂), and the sulfated support contains zirconia (ZrO₂). In some aspects, the noble metal can be palladium (Pd) or platinum (Pt). In some aspects, the group VIII metal can be iron (Fe). In some aspects, the group VIII metal can be iron (Fe) and the group VIII metal oxide can contain a Fe^{III} oxide. In some particular aspects, the Fe^{III} oxide can be Fe₂O₃. In some aspects, the group VI metal can be chromium (Cr). In some aspects, the group VI metal can be chromium (Cr) and the group VI metal oxide can contain a Cr^{III} oxide. In some particular aspects, the chromium III oxide can be Cr₂O₃. In some aspects, the group V metal can be vanadium (V). In some aspects, the group V metal can be vanadium (V) and the group V metal oxide can contain a V^{V} oxide. In some particular aspects, the V^{V} oxide can be V₂O₅.

In some aspects, the supported catalyst composition can contain platinum (Pt) deposited on a sulfated support, the sulfated support can contain zirconia (ZrO₂), Fe present as an oxide, and Cr present as an oxide, wherein the Pt content can be 0.1 to 1 wt. %, Fe content can be 0.5 to 5 wt. %, Cr content can be 0.1 to 2 wt. %, S content can be 1 to 5 wt. %, Zr content can be 50 to 80 wt. % and weight ratio of Pt to Fe can be 1:1 to 1:5 wherein the weight percentages are with respect to the total weight of the catalyst. In some particular aspects, the supported catalyst composition can contain about 0.5 wt. % platinum (Pt) deposited on a sulfated support, the sulfated support can contain zirconia (ZrO₂), about 1.5 wt. % Fe present as an oxide, and about 0.5 wt. % of Cr present as an oxide, wherein the weight percentages are with respect to the total weight of the catalyst. In some particular aspects, the supported catalyst composition can contain platinum (Pt) deposited on a sulfated support, the sulfated support can contain zirconia (ZrO₂), Fe₂O₃, and Cr₂O₃, with Pt content about 0.5 wt. %, Fe₂O₃ content about 1.9 %, Cr₂O₃ content about 0.65 %, sulfate (SO₄²⁻) content about 10 wt. % and optionally the rest of the catalyst being ZrO₂, where the weight percentages are with respect to the total weight of the catalyst.

In some aspects, the supported catalyst composition can contain platinum (Pt) deposited on a sulfated support, the sulfated support can contain zirconia (ZrO₂), Fe present as an oxide, and V present as an oxide, wherein the Pt content can be 0.1 to 1 wt. %, Fe content can be 0.5 to 5 wt. %, V content can be 0.1 to 2 wt. %, S content can be 1 to 5 wt. %, Zr content can be 50 to 80 wt. % and weight ratio of Pt to Fe can be 1:1 to 1:5 wherein the weight percentages are with respect to the total weight of the catalyst. In some particular aspects, the supported catalyst composition can contain about 0.5 wt. % platinum (Pt) deposited on a sulfated support, the sulfated support can contain zirconia (ZrO₂), about 1.5 wt. % Fe present as an oxide; and about 0.5 wt. % of V present as an oxide, wherein the weight percentages are with respect to the total weight of the catalyst. In some particular aspects, the supported catalyst composition can contain platinum (Pt) deposited on a sulfated support, the sulfated support can contain zirconia (ZrO₂), Fe₂O₃, and V₂O₅, with Pt content about 0.5 wt. %, Fe₂O₃ content about 1.9 %, V₂O₅ content about 0.75 %, sulfate (SO₄²⁻) content about 10 wt. % and optionally the rest of the catalyst being ZrO₂, where the weight percentages are with respect to the total weight of the catalyst.

In some aspects, the supported catalyst composition can contain palladium (Pd) deposited on a sulfated support, the sulfated support can contain zirconia (ZrO₂), Fe present as an oxide, and Cr present as an oxide, wherein the Pd content can be 0.1 to 1 wt. %, Fe content can be 0.5 to 5 wt. %, Cr content can be 0.1 to 2 wt. %, S content can be 1 to 5 wt. %, Zr content can be 50 to 80 wt. % and weight ratio of Pd to Fe can be 1:1 to 1:5 wherein the weight percentages are with respect to the total weight of the catalyst. In some particular aspects, the supported catalyst composition can contain about 0.5 wt. % palladium (Pd) deposited on a sulfated support, the sulfated support can contain zirconia (ZrO₂), about 1.5 wt. % iron Fe present as an oxide, and about 0.5 wt. % of Cr present as an oxide, wherein the weight percentages are with respect to the total weight of the catalyst. In some particular aspects, the supported catalyst composition can contain palladium (Pd) deposited on a sulfated support, the sulfated support can contain zirconia (ZrO₂), Fe₂O₃, and Cr₂O₃, with Pd content about 0.5 wt. %, Fe₂O₃ content about 1.9 %, Cr₂O₃ content about 0.65 %, sulfate (SO₄²⁻) content about 10 wt. % and optionally the rest of the catalyst being ZrO₂, where the weight percentages are with respect to the total weight of the catalyst.

In some aspects, the supported catalyst composition can contain palladium (Pd) deposited on a sulfated support, the sulfated support can contain zirconia (ZrO₂), Fe present as an oxide, and V present as an oxide, wherein the Pd content can be 0.1 to 1 wt. %, Fe content can be 0.5 to 5 wt. %, V content can be 0.1 to 2 wt. %, S content can be 1 to 5 wt. %, Zr content can be 50 to 80 wt. % and weight ratio of Pd to Fe can be 1:1 to 1:5 wherein the weight percentages are with respect to the total weight of the catalyst. In some aspects, the supported catalyst composition can contain about 0.5 wt. % palladium (Pd) deposited on a sulfated support, the sulfated support can contain zirconia (ZrO₂), about 1.5 wt. % Fe present as an oxide; and about 0.5 wt. % of V present as an oxide, wherein the weight percentages are with respect to the total weight of the catalyst. In some particular aspects, the supported catalyst composition can contain palladium (Pd) deposited on a sulfated support, the sulfated support can contain zirconia (ZrO₂), Fe₂O₃; and V₂O₅, with Pd content about 0.5 wt. %, Fe₂O₃ content about 1.9 %, V₂O₅ content about 0.75 %, sulfate (SO₄²⁻) content about 10 wt. % and optionally the rest of the catalyst being ZrO₂, where the weight percentages are with respect to the total weight of the catalyst.

One aspect of the present invention is directed to a process for preparing a supported catalyst composition as described herein. The supported catalyst composition can be used for catalyzing isomerization of C4-C7 n-paraffin hydrocarbons. The process can include any one of, any combination of, or all of steps (a) to (j). In step (a), an aqueous solution containing a group IV element salt and/or an aluminum salt, a group VIII metal salt, and a group V metal salt and/or a group VI metal salt can be prepared. In some aspects, the aqueous solution in step (a) can contain a group IV element salt, a group VIII metal salt and a group V metal salt. In some aspects, the aqueous solution in step (a) can contain a group IV element salt, a group VIII metal salt and a group VI metal salt. In some aspects, a precipitating agent can added to the aqueous solution to form a precipitate. In some particular aspects, the precipitating agent can be an alkali solution. In some particular aspects, the alkali solution can be ammonium hydroxide solution. In some particular aspects, the alkali solution can be ammonium hydroxide solution with ammonium hydroxide content 5 wt. % to 25 wt. %, or 10 wt. % to 20 wt. % or 12 wt. % to 18 wt. % or about 15 wt. %. In step (b), the precipitate can be collected from the aqueous solution, where the precipitate can contain hydroxide(s) of the group IV element and/or of aluminum, hydroxide(s) of the of the group VIII metal, and hydroxide(s) of the of the group V metal and/or of the group VI metal. In some aspects, the precipitate in step (b) can contain hydroxide(s) of the group IV element, hydroxide(s) of the group VIII metal and hydroxide(s) of the group V metal. In some aspects, the precipitate in step (b) can contain hydroxide(s) of the group IV element, hydroxide(s) of the group VIII metal and hydroxide(s) of the group VI metal. In step (c) the precipitate can be washed to remove chloride ions and to obtain a wet cake; In step (d), the wet cake can be dried to obtain a dried support. In step (e), the dried support can be added to a sulfate containing solution to obtain a sulfate soaked support. In step (f), the sulfate soaked support can be separated from the sulfate containing solution and dried to obtain a sulfated dried support. In step (g), the sulfated dried support can be calcined to obtain a calcined support. In some aspects, the dried support can be calcined in presence of air, at a temperature between 550 °C to 750 °C for 1 hour to 30 hours. In step (h), a solution containing a noble metal salt can be added to the calcined support to obtain an impregnated support e.g. metal loaded support. In step (i), the impregnated support e.g. metal loaded support can be dried to obtain a dried impregnated support e.g. dried metal loaded support. In step (j), the dried impregnated support e.g. metal loaded support can be calcined to obtain the supported catalyst composition. In some aspects, the dried impregnated support e.g. dried metal loaded support can be calcined in presence of air at a temperature 350 °C to 650 °C for 1 hour to 30 hours.

Another aspect of the present invention is directed to, a process for isomerizing a C4-C7 n-paraffin hydrocarbon. The process can include contacting a reactant stream containing a C4-C7 n-paraffin hydrocarbon with a supported catalyst composition in presence of hydrogen (H₂), under conditions suitable to isomerize at least a portion of the C4-C7 n-paraffin hydrocarbon and produce a products stream containing a C4-C7 iso-paraffin hydrocarbon. The contacting and/or reaction conditions can include a temperature between 100 °C to 350 °C, preferably between 150 °C to 250 °C; a pressure between 100 kPa to 50 MPa, preferably between 10 MPa to 30 MPa; LHSV between 0.25 h⁻¹ to 30 h⁻¹, preferably 5 h⁻¹ to 20 h⁻¹; a hydrogen to C4-C7 n-paraffin hydrocarbons molar ratio between 0.05:1 0.5:1, 1:1, 1.5:1, 2:1, 2.5:1, 3:1, 3.5:1, 4:1, 4.5:1 to 5:1, preferably between 0.1:1 to 1:1; or a combination thereof. In some aspects, the C4-C7 n-paraffin hydrocarbon is n-butane and C4-C7 iso-paraffin hydrocarbon is isobutane. In some aspects, the C4-C7 n-paraffin hydrocarbon is n-pentane and C4-C7 iso-paraffin hydrocarbon is isopentane. In some aspects, the C4-C7 n-paraffin hydrocarbon is n-hexane and the C4-C7 iso-paraffin hydrocarbon is 2,2-dimethylpropane, 2-methylpentane, 3-methylpentane, 2,2-dimethylbutane, 2,3-dimethylbutane, or a combination thereof. In some aspects, the C4-C7 n-paraffin hydrocarbon is n-heptane and the C4-C7 iso-paraffin hydrocarbon is 2-methylhexane, 3-methylhexane, 2,2-dimethylpentane, 2,3-dimethylpentane, 2,4-dimethylpentane, 3,3-dimethylpentane, 3-ethylpentane or a combination thereof. The some embodiments the reactant stream can contain a mixture of C4-C7 n-paraffin hydrocarbons.

Other embodiments of the invention are discussed throughout this application. Any embodiment discussed with respect to one aspect of the invention applies to other aspects of the invention as well and vice versa. Each embodiment described herein is understood to be embodiments of the invention that are applicable to other aspects of the invention. It is contemplated that any embodiment discussed herein can be implemented with respect to any method or composition of the invention, and vice versa. Furthermore, compositions and systems of the invention can be used to achieve methods of the invention.

The following includes definitions of various terms and phrases used throughout this specification.

C4-C7 n-paraffin hydrocarbon refers a linear paraffin hydrocarbons with a carbon number between 4 to 7 (e.g. n-butane, n-pentane, n-hexane, n-heptane etc.).

C4-C7 iso-paraffin hydrocarbon refers a branched paraffin hydrocarbons with a carbon number between 4 to 7 (e.g. isobutane, isopentane, 2,2-dimethylpropane, 2-methylpentane, 3-methylpentane, 2,2-dimethylbutane, 2,3-dimethylbutane, 2-methylhexane, 3-methylhexane, 2,2-dimethylpentane, 2,3-dimethylpentane, 2,4-dimethylpentane, 3,3-dimethylpentane, 3-ethylpentane etc.).

Phrases "isomerizing a C4-C7 n-paraffin hydrocarbon" or "isomerization of C4-C7 n-paraffin hydrocarbon" refer to formation of one or more C4-C7 iso-paraffin hydrocarbon(s) from a C4-C7 n-paraffin hydrocarbon having the same carbon number.

The terms "about" or "approximately" are defined as being close to as understood by one of ordinary skill in the art. In one non-limiting embodiment, the terms are defined to be within 10%, preferably within 5%, more preferably within 1%, and most preferably within 0.5%.

The terms "wt.%," "vol.%," or "mol.%" refers to a weight percentage of a component, a volume percentage of a component, or molar percentage of a component, respectively, based on the total weight, the total volume of material, or total moles, that includes the component. In a non-limiting example, 10 grams of component in 100 grams of the material is 10 wt.% of component.

The term "substantially" and its variations are defined to include ranges within 10%, within 5%, within 1%, or within 0.5%. "Essentially free" is defined as having no more than about 0.1% of a component.

The terms "inhibiting" or "reducing" or "preventing" or "avoiding" or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the words "a" or "an" when used in conjunction with any of the terms "comprising," "including," "containing," or "having" in the claims, or the specification, may mean "one," but it is also consistent with the meaning of "one or more," "at least one," and "one or more than one." The phrase "and/or" means and or or. To illustrate, A, B, and/or C includes: A alone, B alone, C alone, a combination of A and B, a combination of A and C, a combination of B and C, or a combination of A, B, and C. In other words, "and/or" operates as an inclusive or.

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The process and systems of the present invention can "comprise," "consist essentially of," or "consist of" particular ingredients, components, compositions, steps, *etc.* disclosed throughout the specification. With respect to the transitional phrase "consisting essentially of," in one non-limiting aspect, a basic and novel characteristic of the compositions and processes of the present invention are supported catalyst compositions having the ability to produce C4-C7 iso-paraffin hydrocarbons by isomerization of C4-C7 n-paraffin hydrocarbons.

Other objects, features and advantages of the present invention will become apparent from the following figures, detailed description, and examples. It should be understood, however, that the figures, detailed description, and examples, while indicating specific embodiments of the invention, are given by way of illustration only and are not meant to be limiting. Additionally, it is contemplated that changes and modifications within the spirit and scope of the invention will become apparent to those skilled in the art from this detailed description. In further embodiments, features from specific embodiments may be combined with features from other embodiments. For example, features from one embodiment may be combined with features from any of the other embodiments. In further embodiments, additional features may be added to the specific embodiments described herein.

### DETAILED DESCRIPTION OF THE INVENTION

A discovery has been made that provides a solution to at least some of the aforementioned problems associated with catalysts used for isomerization of n-paraffin hydrocarbons to produce iso-paraffin hydrocarbons. The solution is premised on developing a supported catalyst composition containing a noble metal and a sulfated support. The sulfated support can include an oxide or hydroxide of a group IV element and/or of aluminum, a group VIII metal oxide and a group V metal oxide and/or a group VI metal oxide. The weight ratio of the noble metal to the group VIII metal can be 1:1 to 1:5. It was surprisingly found that the supported catalyst compositions provide a good conversion percentage, selectivity, and yield during isomerization n-paraffin hydrocarbon to iso-paraffin hydrocarbon. By way of example, and as illustrated in a non-limiting manner in the Examples, the conversion, selectivity, and yield for n-butane to iso-butane isomerization using such catalysts can be between 20, 25, 30, 35, 40, 45 % to 50 %; 75, 80, 85 % to 95 %; and 18, 20, 25, 30, 35 % to 40 % respectively.

These and other non-limiting aspects of the present invention are discussed in the following sections.

### A. Supported Catalyst Composition

In one aspect of the present invention, a supported catalyst composition is described. The supported catalyst composition can be used for catalyzing isomerization of C4-C7 n-paraffin hydrocarbons to form C4-C7 iso-paraffin hydrocarbons. The supported catalyst composition can contain a noble metal and a sulfated support. The sulfated support can include an oxide or hydroxide of a group IV element and/or of aluminum, a group VIII metal oxide and a group V metal oxide and/or a group VI metal oxide, wherein weight ratio of the noble metal to the group VIII metal is between 1:1 to 1:5 or at least any one of, equal to any one of, or between any two of 1:1, 1:1.5, 1:2, 1:2.5, 1:3, 1:3.5, 1:4, 1:4.5 and 1:5. While not intended to be limited by theory, it is believed the noble metal functions as a catalytically active component and the group VIII metal oxide, the group V metal oxide, and/or the group VI metal oxide functions as promoters of the supported catalyst composition. The noble metal can be platinum (Pt), palladium (Pd) ruthenium (Ru), rhodium (Rh), silver (Ag), osmium (Os), iridium (Ir), and/or gold (Au); preferably platinum (Pt), and/or palladium (Pd). The group IV element can be zirconium (Zr), titanium (Ti), hafnium (Hf), tin (Sn), lead (Pb), silicon (Si), and/or germanium (Ge); preferably zirconium (Zr), titanium (Ti), and/or silicon (Si); more preferably zirconium (Zr). The group IV element oxide or hydroxide can be zirconium hydroxide (Zr(OH)₄), zirconia (ZrO₂), silica (SiO₂), and/or titania (TiO₂), preferably zirconia (ZrO₂). The group VIII element e.g. metal can be iron (Fe), cobalt (Co), and/or nickel (Ni); preferably iron (Fe). In some aspects, the group VIII metal oxide can be an iron (Fe) oxide. In some particular aspect, the iron oxide can contain an Fe^{III} oxide. In some particular aspect, the Fe^{III} oxide can be Fe₂O₃. The group V element e.g. metal can be vanadium (V), niobium (Nb), antimony (Sb), bismuth (Bi), and/or tantalum (Ta); preferably vanadium (V). In some aspects the group V metal oxide can be a vanadium (V) oxide. In some particular aspect, the vanadium oxide can contain an V^{V} oxide. In some particular aspect, the V^{V} oxide can be V₂O₅. The group VI metal can be chromium (Cr), molybdenum (Mo), tungsten (W), and/or technetium (Te); preferably chromium (Cr), In some aspects the group VI metal oxide can be an chromium (Cr) oxide. In some particular aspect, the chromium oxide can contain a Cr^{III} oxide. In some particular aspect, the Cr^{III} oxide can be Cr₂O₃. In some aspects, the composition can contain 0.1 wt. % to 1 wt. % or at least any one of, equal to any one of, or between any two of 0.1 wt. %, 0.2 wt. %, 0.3 wt. %, 0.4 wt. %, 0.5 wt. %, 0.6 wt. %, 0.7 wt. %, 0.8 wt. %, 0.9 wt. % and 1 wt. % of the noble metal. In some aspects, the composition can contain 0.5 wt. % to 5 wt. % or at least any one of, equal to any one of, or between any two of 0.5 wt. %, 1.0 wt. %, 1.5 wt. %, 2.0 wt. %, 2.5 wt. %, 3.0 wt. %, 3.5 wt. %, 4.0 wt. %, 4.5 wt. %, and 5 wt. % of the group VIII metal. In some aspects, the composition can contain 0.1 wt. % to 2 wt. % or at least any one of, equal to any one of, or between any two of 0.1 wt. %, 0.2 wt. %, 0.4 wt. %, 0.6 wt. %, 0.8 wt. %, 1.0 wt. %, 1.2 wt. %, 1.4 wt. %, 1.6 wt. %, 1.8 wt. %, and 2 wt. % of the group V metal. In some aspects, the composition can contain 0.1 wt. % to 2 wt. % or at least any one of, equal to any one of, or between any two of 0.1 wt. %, 0.2 wt. %, 0.4 wt. %, 0.6 wt. %, 0.8 wt. %, 1.0 wt. %, 1.2 wt. %, 1.4 wt. %, 1.6 wt. %, 1.8 wt. %, and 2 wt. % of the group VI metal. In some aspects, the composition can contain 50 wt. % to 80 wt. % or at least any one of, equal to any one of, or between any two of 50 wt. %, 55 wt. %, 60 wt. %, 65 wt. %, 70 wt. %, 75 wt. %, and 80 wt. %, of the group IV element. In some aspects, the composition can contain 1 wt. % to 5 wt. % or at least any one of, equal to any one of, or between any two of 1 wt. %, 1.5 wt. %, 2.0 wt. %, 2.5 wt. %, 3.0 wt. %, 3.5 wt. %, 4.0 wt. %, 4.5 wt. %, and 5 wt. % of sulfur, present as sulfate. In some aspects, weight ratio of the noble metal to the group VI metal can be between 1:0.5 to 1:1.5 or at least any one of, equal to any one of, or between any two of 1:0.5, 1:0.6, 1:0.7, 1:0.8, 1:0.9, 1:1, 1:1.1, 1:1.2, 1:1.3, 1:1.4 and 1:1.5. In some aspects, weight ratio of the noble metal to the group V metal can be between 1:0.1 to 1:1.2 or at least any one of, equal to any one of, or between any two of 1:0.1, 1:0.2, 1:0.3, 1:0.4, 1:0.5, 1:0.6, 1:0.7, 1:0.8, 1:0.9, 1:1, 1:1.1, and 1:1.2. In some aspects, weight ratio of the group VI metal to the group VIII metal can be between 1:1 to 1:5 or at least any one of, equal to any one of, or between any two of 1:1, 1:1.5, 1:2, 1:2.5, 1:3, 1:3.5, 1:4, 1:4.5 and 1:5. In some aspects, weight ratio of the group V metal to the group VIII metal can be between 1:2 to 1:10 or at least any one of, equal to any one of, or between any two of 1:2, 1:2.5, 1:3, 1:3.5, 1:4, 1:4.5, 1:5, 1:5.5, 1:6, 1:6.5, 1:7, 1:7.5, 1:8, 1:8.5, 1:9, 1:9.5 and 1:10. In some aspects, the chlorine content of the catalyst composition can be below 1, 0.5, 0.1, or 0 wt. %. In some aspects, the chlorine content of the catalyst composition can be 0 wt. % to 0.1 wt. %. In some aspects, the catalyst composition can be essentially free of manganese (Mn).

In some aspects, the supported catalyst composition of the current invention can have a surface area 70 m²/g to 200 m²/g or at least any one of, equal to any one of, or between any two of 70 m²/g, 80 m²/g, 90 m²/g, 100 m²/g, 110 m²/g, 120 m²/g, 130 m²/g, 140 m²/g, 150 m²/g, 160 m²/g, 170 m²/g, 180 m²/g, 190 m²/g, and 200 m²/g. In some aspects, the supported catalyst composition of the current invention can have a pore size 2 nm to 10 nm or at least any one of, equal to any one of, or between any two of, 2 nm, 3 nm, 4 nm, 5 nm, 6 nm, 7 nm, 8 nm, 9 nm, and 10 nm.

### B. Methods of Making a Support Catalyst Composition

In one aspect of the present invention, a method to prepare a supported catalyst composition is described. The supported catalyst composition can be prepared using the following steps. In some aspects, a group VIII metal and a group VI metal and/or a group V metal promoted group IV element hydroxide can be prepared by a co-precipitation method. In some aspects, a group VIII metal and a group VI metal and/or a group V metal promoted group IV element and/or aluminum hydroxide can be prepared by co-precipitation method. A group VI element salt and/or aluminum salt, a group VIII metal salt, and a group VI metal salt and/or group V metal salt, can be dissolved in water to prepare an aqueous solution. In some aspects, the group IV element can be zirconium (Zr) and the group IV element salt can be zirconyl chloride (ZrOCl₂), zirconyl chloride octahydrate (ZrOCl₂.8H₂O), and/or zirconyl nitrate hydrate (ZrO(NO₃)₂.xH₂O. In some aspects, the group VIII metal can be iron (Fe) and the group VIII metal salt can be, ferric nitrate (Fe(NO₃)₃), ferric nitrate nonahydrate (Fe(NO₃)₃.9H₂O), ferric chloride (FeCl₃), ferric chloride hexahydrate (FeCl₃.6H₂O), and/or ferric sulfate hydrate (Fe₂(SO₄)₃.xH₂O), In some aspects, the group VI metal can be chromium (Cr) and the group VI metal salt can be, chromium nitrate (Cr(NO₃)₃), chromium nitrate nonahydrate (Cr(NO₃)₃.9H₂O), chromium chloride hexahydrate (CrCl₃.6H₂O), chromic acid (H₂CrO₄), and/or chromium trioxide (CrO₃). In some aspects, the group V metal can be vanadium (V) and the group V metal salt can be, vanadyl oxalate (VOC₂O₄), vanadyl oxalate dihydrate (VOC₂O₄.2H₂O), and/or ammonium metalvanadate (NH₄VO₃). In some aspects, the water can be demineralized water. An alkali solution can be added to the aqueous solution to obtain a precipitate containing hydroxide(s) of the group IV element and/or of aluminum, hydroxide(s) of the group VIII metal, and hydroxide(s) of the group V metal and/or a group VI metal from the aqueous solution. In some aspects, the alkali solution can be an ammonium hydroxide (NH₄OH) solution and an ammonium hydroxide solution can be added to the aqueous solution under stirring until the pH of the resulting solution reached 7.0 or above, such as about 7, 7.5, 8, 8.5 or 8.71 to obtain the precipitate. The precipitate can be recovered and washed to remove chloride ions and obtain a wet cake. In some aspects, the precipitate can be recovered via vacuum filtration and washed with water, such as deionized water to remove chloride ions and decrease pH to about 7.0 to obtain the wet cake. The wet cake can be dried to obtain a dried support. In some aspects, the wet cake can be dried at a temperature between 100 °C to 160 °C or at least any one of, equal to any one of, or between any two of 100 °C, 110 °C, 120 °C, 130 °C, 140 °C, 150 °C, and 160 °C for 2 h to 48 h or at least any one of, equal to any one of, or between any two of 2 h, 4 h, 6 h, 8 h, 10 h, 12 h, 14 h, 16 h, 18 h, 20 h, 22 h, 24 h, 26 h, 28 h, 30 h, 32 h, 34 h, 36 h, 38 h, 40 h, 42 h, 44 h, 46 h, and 48 h to obtain the dried support. In some aspects, the dried support can be grounded in to fine powder. The dried support or the grounded dried support can be added to a sulfate solution to obtain a sulfate soaked support. In some aspects the sulfate solution can be a sulfuric acid solution, and/or ammonium sulfate solution; preferably a sulfuric acid solution. The sulfate soaked support can be dried to obtain a sulfated dried support. In some aspects, the sulfated soaked support can be filtered by vacuum filtration without washing and the obtained material can be dried at a temperature between 100 °C to 160 °C or at least any one of, equal to any one of, or between any two of 100 °C, 110 °C, 120 °C, 130 °C, 140 °C, 150 °C, and 160 °C for 2 h to 48 h or at least any one of, equal to any one of, or between any two of 2 h, 4 h, 6 h, 8 h, 10 h, 12 h, 14 h, 16 h, 18 h, 20 h, 22 h, 24 h, 26 h, 28 h, 30 h, 32 h, 34 h, 36 h, 38 h, 40 h, 42 h, 44 h, 46 h, and 48 h to obtain the sulfated dried support. The sulfated dried support can be calcined to obtain a calcined support. In some aspects, the sulfated dried support can be calcined in presence of air, at a temperature between 550 °C to 750 °C or at least any one of, equal to any one of, or between any two of 550 °C, 575 °C, 600 °C, 625 °C, 650 °C, 675 °C, 700 °C, 725 °C, and 750 °C for 1 h to 30 h or at least any one of, equal to any one of, or between any two of 1 h, 5 h, 10 h, 15 h, 20 h, 25 h and 30 h to obtain the calcined support. In some aspects, the sulfated dried support can be calcined in a muffle furnace at 600°C for 4 h with heating rate 2°C/min in air flow (100 ml/min) to obtain the calcined support. The calcined support can be impregnated with a noble metal salt to obtain an impregnated support e.g. metal loaded support. Alternatively, the noble metal can be deposited on the surface of the calcined support (e.g., by precipitation-deposition, reduction-deposition, metal vapor deposition etc.) to obtain the metal loaded support. With respect to impregnation, and in some aspects, the noble metal is platinum (Pt) and the noble metal salt can be hexachloroplatinic acid (H₂PtCl₆), platinum chloride (PtCl₄), ammonium chlorplatinate ((NH₄)₂[PtCl₆]), tetraamineplatinum nitrate ((Pt(NH₃)₄(NO₃)₂), and/ or platinum nitrate (Pt(NO₃)₄). In some aspects, the noble metal is palladium (Pd) and the noble metal salt can be palladium chloride (PdCl₂), and/or palladium nitrate (Pd(NO₃)₂). The impregnated support e.g. metal loaded support can be dried to obtain a dried impregnated support e.g. dried metal loaded support. In some aspects, the impregnated support e.g. metal loaded support can be dried at a temperature between 100 °C to 160 °C or at least any one of, equal to any one of, or between any two of 100 °C, 110 °C, 120 °C, 130 °C, 140 °C, 150 °C, and 160 °C for 2 h to 48 h or at least any one of, equal to any one of, or between any two of 2 h, 4 h, 6 h, 8 h, 10 h, 12 h, 14 h, 16 h, 18 h, 20 h, 22 h, 24 h, 26 h, 28 h, 30 h, 32 h, 34 h, 36 h, 38 h, 40 h, 42 h, 44 h, 46 h, and 48 h to obtain the dried impregnated support e.g. dried metal loaded support. In some aspects, the impregnated support e.g. metal loaded support can be allowed to dry at room temperature for 16 h and dried at 120°C for 16 h in air-oven to obtain the dried impregnated support e.g. dried metal loaded support. The dried impregnated support e.g. dried metal loaded support can be fine powered and calcined to obtain the supported catalyst composition. In some embodiments, the dried impregnated support e.g. dried metal loaded support or fine powdered dried impregnated support e.g. dried metal loaded support can be calcined in presence of air, at a temperature between 350 °C to 650 °C or at least any one of, equal to any one of, or between any two of 350 °C, 375 °C, 400 °C, 425 °C, 450 °C, 475°C, 500 °C, 525 °C, 550 °C, 575°C, 600 °C, 625 °C, and 650 °C for 1 h to 30 h or at least any one of, equal to any one of, or between any two of 1 h, 5 h, 10 h, 15 h, 20 h, 25 h and 30 h to obtain the supported catalyst composition. In some aspects, the dried impregnated support e.g. dried metal loaded support can be fine powdered and calcined in a muffle furnace at 550 °C for 2 h with heating rate 2°C/min in air flow (100 ml/min) to obtain the supported catalyst composition. The amounts of the group IV element salt, aluminum salt, group VIII metal salt, group V metal salt, group VI metal salt, and sulfate used during the process of preparation of the supported catalyst composition can be adjusted such that the supported catalyst composition contains 0.1 wt. % to 1 wt. % of the noble metal, 0.5 wt. % to 5 wt. % of the group VIII metal, 1 wt. % to 5 wt. % of sulfur as sulfate (SO₄²⁻), 50 wt. % to 80 wt. % of the group IV element and/or aluminum, and 0.1 wt. % to 2 wt. % of the group V metal and/or 0.1 wt. % to 2 wt. % of the group VI metal, with weight ratio of the noble metal to the group VIII metal is 1:1 to 1:5.

### C. Methods of Using the Supported Catalyst Composition

The supported catalyst compositions can be used for isomerization of n-paraffin hydrocarbons to produce iso-paraffin hydrocarbons. A reactant stream containing a C4-C7 n-paraffin hydrocarbon can be contacted with the supported catalyst composition in presence of hydrogen (H₂), under conditions suitable to isomerize at least a portion of the C4-C7 n-paraffin hydrocarbon and produce a products stream containing a C4-C7 iso-paraffin hydrocarbon. The contacting conditions can include a temperature between 100 °C to 350 °C, or at least any one of, equal to any one of, or between any two of 100 °C, 125 °C, 150 °C, 175 °C, 200 °C, 225°C, 250 °C, 275 °C, 300 °C, 325°C, and 350 °C, a pressure between 100 kPa to 50 MPa or at least any one of, equal to any one of, or between any two of 100 kPa, 1 MPa, 10 MPa, 20 MPa, 30 MPa, 40 MPa, and 50 MPa, liquid hourly space velocity (LHSV) between 0.25 h⁻¹ to 30 h⁻¹ or at least any one of, equal to any one of, or between any two of 0.25 h⁻¹, 1 h⁻¹, 5 h⁻¹, 10 h⁻¹, 15 h⁻¹, 20 h⁻¹, 25 h⁻¹, 30 h⁻¹, 35 h⁻¹, 40 h⁻¹, 45 h⁻¹, and 50 h⁻¹, or combination thereof. H₂ to C4-C7 n-paraffin hydrocarbons molar ratio can be between 0.05:1 to 5:1, or at least any one of, equal to any one of, or between any two of 0.05:1, 0.1:1, 0.3:1, 0.5:1, 0.7:1, 0.9:1, 1:1, 2:1, 3:1, 4:1 and 5:1. In some aspects, the supported catalyst composition can be reduced prior to contacting the supported catalyst composition with the reactant stream. In some aspects, the C4-C7 n-paraffin hydrocarbon is n-butane and C4-C7 iso-paraffin hydrocarbon is iso butane. In some aspects, n-butane conversion in n-butane to iso-butane isomerization reaction using the supported catalyst composition of the current invention can be between 20 % to 50 %, or at least any one of, equal to any one of, or between any two of 20 %, 25 %, 30 %, 35 %, 40 %, 45 %, and 50 %. In some aspects, iso-butane selectivity in n-butane to iso-butane isomerization reaction using the supported catalyst composition of the current invention can be between 75 % to 95 %, or at least any one of, equal to any one of, or between any two of 75 %, 77 %, 79 %, 81 %, 83 %, 85 %, 87 %, 89 %, 91 %, 93 %,and 95 %. In some aspects, n-butane to iso-butane isomerization reaction yield using the supported catalyst composition of the current invention can be between 18 % to 40 %, or at least any one of, equal to any one of, or between any two of 18 %, 20 %, 22 %, 24 %, 26 %, 28 %, 30 %, 32 %, 34 %, 36 %, 38 %, and 40 %.

Isomerization of C4-C7 n-paraffin hydrocarbon can be performed in fixed bed or fluidized bed reactors (e.g., a circulating bubbling fluidized bed, a circulating turbulent fluidized bed, a fast fluidized bed, ebullated bed, a dense phase riser or a dilute phase riser). In some aspects, the supported catalyst can be fluidized in or with the reactant stream. In some aspects, the supported catalyst can be positioned in a fixed bed. When positioned in a fixed bed, contacting the reactant stream with the supported catalyst can include passing the reactant stream over or through the fixed bed. In some aspects, the reactor can include one or more heating and/or cooling devices (e.g., insulation, electrical heaters, jacketed heat exchangers in the wall) and/or controllers (e.g., computers, flow valves, automated values, inlets, outlets, *etc*.) that can be used to control the reaction temperature and pressure of the reaction mixture. A single or multiple reactors can be used. The reactors can be positioned parallel and/or in series.

### EXAMPLES

As part of the disclosure of the present invention, specific examples are included below. The examples are for illustrative purposes only and are not intended to limit the invention. Those of ordinary skill in the art will readily recognize parameters that can be changed or modified to yield essentially the same results.

### Example 1

### Catalyst preparation

### Comparative catalyst A: Pd/SO₄²⁻/Fe₂O₃-ZrO₂

Iron promoted zirconium hydroxide (Fe-Zr(OH)ₓ, where x balances the charge of the metals) was prepared by co-precipitation method. 130 g of zirconyl chloride octahydrate (ZrOCl₂.8H₂O) and 7.1929 g of iron nitrate nonahydrate (Fe(NO₃)₃.9H₂O) was dissolved in 975 ml of DM water. To above solution, added dropwise (30 - 40 min) 15 wt.% ammonium hydroxide solution (NH₄OH) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing) forming a wet material. The obtained wet material was dried at 120 °C for 16 h in air-oven. The obtained dried material was calcined in muffle furnace at 600 °C for 4 h with heating rate 2 °C/min in air flow (100 ml/min).

Palladium supported catalyst was prepared via incipient wetness impregnation method. To 44.775 g of calcined support added dropwise metal solution. The Pd solution was prepared according to this procedure: 0.3749 g PdCl₂ (0.5 wt% Pd) was added to 10 ml of DM water (based on pore volume of support: 0.22 ml/g) and also added 3 drops of 37% HCl solution. The impregnated catalyst material was allowed to dry at room temperature for 16 h and dried at 120° C for 16 h in air-oven. The dried material was fine powdered and calcined in muffle furnace at 400 °C for 2 h with heating rate 2 °C/min in air flow (100 ml/min). The final calculated composition of the catalyst corresponds to Pd - 0.5 wt. %, Fe₂O₃ -2.9 wt %, SO₄²⁻ -10 wt. % with remaining part comprising ZrO₂.

### Comparative Catalyst B: Pd/SO₄²⁻/Cr₂O₃-ZrO₂

Chromium promoted zirconium hydroxide (Cr-Zr(OH)ₓ, where x balances the charge of the metals) was prepared by co-precipitation method. 130 g of zirconyl chloride octahydrate (ZrOCl₂.8H₂O) and 7.5608 g of chromium nitrate nonahydrate (Cr(NO₃)₃.9H₂O) (7.5608 g) was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered chromium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min). Palladium supported catalyst was prepared from the calcined support as the procedure described for comparative catalyst A. The final calculated composition of the catalyst corresponds to Pd - 0.5 wt. %, Cr₂O₃ -2.8 wt%, SO₄²⁻ - 10 wt. % with remaining part comprising ZrO₂.

### Comparative Catalyst C: Pd/SO₄²⁻/V₂O₅-ZrO₂

Vanadium promoted zirconium hydroxide (V-Zr(OH)ₓ, where x balances the charge of the metals) was prepared by co-precipitation method. 130 g of zirconyl chloride octahydrate (ZrOCl₂.8H₂O) and 3.7656 g of vanadyl oxalate dihydrate (VOC₂O₄.2H₂O) was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered vanadium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min). Palladium supported catalyst was prepared from the calcined support as the procedure described for comparative catalyst A. The final calculated composition of the catalyst corresponds to Pd - 0.5 wt. %, V₂O₅ -3.0 wt%, SO₄²⁻ -10 wt. % with remaining part comprising ZrO₂.

### Comparative Catalyst D: Pd/SO₄²⁻/Fe₂O₃-Mn₂O₃-ZrO₂

Iron and manganese promoted zirconium hydroxide (Fe-Mn-Zr(OH)ₓ, where x balances the charge of the metals) was prepared by co-precipitation method. 130 g of zirconyl chloride octahydrate (ZrOCl₂. 8H₂O), 5.39 g of iron nitrate nonahydrate (Fe(NO₃)₃.9H₂O) and 1.13 g of manganese nitrate tetrahydrate (Mn(NO₃)₂.4H₂O) (1.13 g) was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120°C for 16 h and grounded in to fine powder. For sulfation, the dried powdered iron-manganese-zirconium hydroxide material was added to 300 ml (292.5 ml H₂O + 8.125 ml 98%H2SO4) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min). Palladium supported catalyst was prepared from the calcined support as the procedure described for comparative catalyst A. The final calculated composition of the catalyst corresponds to Pd - 0.5 wt. %, Fe₂O₃ -1.9 wt. %, Mn₂O₃ - 0.7 wt. %, SO₄²⁻ -10 wt. % with remaining part comprising ZrO₂.

### Catalyst 1-A: Pd/SO₄²⁻/Fe₂O₃-Cr₂O₃-ZrO₂

Iron and chromium promoted zirconium hydroxide (Fe-Cr-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 1.9127 g of chromium nitrate tetrahydrate Cr(NO₃)₃.9H₂O was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-chromium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min).

Palladium supported catalyst was prepared via incipient wetness impregnation method. To 44.775 g of calcined support added dropwise metal solution. The Pd solution was prepared according to this procedure: 0.3749 g PdCl₂ (0.5wt% Pd) was added to 10 ml of DM water (based on pore volume of support: 0.22 ml/g) and also added 3 drops of 37% HCl solution. The impregnated catalyst material was allowed to dry at room temperature for 16 h and dried at 120 °C for 16 h in air-oven. The dried material was fine powdered and calcined in muffle furnace at 400 °C for 2 h with heating rate 2 °C/min in air flow (100 ml/min).

The final calculated composition of the catalyst corresponds to Pd - 0.5 wt. %, Fe₂O₃ - 1.9 wt. %, Cr - 0.65 wt. %, SO₄²⁻ - 10 wt. % with remaining part comprising ZrO₂.

### Catalyst 1-B: Pd/SO₄²⁻/Fe₂O₃-Cr₂O₃-ZrO₂

Iron and chromium promoted zirconium hydroxide (Fe-Cr-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 1.9127 g of chromium nitrate tetrahydrate Cr(NO₃)₃.9H₂O was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-chromium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min).

Palladium supported catalyst was prepared via incipient wetness impregnation method. To 44.775 g of calcined support added dropwise metal solution. The Pd solution was prepared according to this procedure: 0.3749 g PdCl₂ (0.5wt% Pd) was added to 10 ml of DM water (based on pore volume of support: 0.22 ml/g) and also added 3 drops of 37% HCl solution. The impregnated catalyst material was allowed to dry at room temperature for 16 h and dried at 120 °C for 16 h in air-oven. The dried material was fine powdered and calcined in muffle furnace at 450 °C for 2 h with heating rate 2 °C/min in air flow (100 ml/min).

The final calculated composition of the catalyst corresponds to Pd - 0.5 wt. %, Fe₂O₃ - 1.9 wt. %, Cr-0.65 wt.%, SO₄²⁻ - 10 wt. % with remaining part comprising ZrO₂.

### Catalyst 1-C: Pd/SO₄²⁻/Fe₂O₃-Cr₂O₃-ZrO₂

Iron and chromium promoted zirconium hydroxide (Fe-Cr-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 1.9127 g of chromium nitrate tetrahydrate Cr(NO₃)₃.9H₂O was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-chromium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2° C/min in air flow (100 ml/min).

Palladium supported catalyst was prepared via incipient wetness impregnation method. To 44.775 g of calcined support added dropwise metal solution. The Pd solution was prepared according to this procedure: 0.3749 g PdCl₂ (0.5 wt. % Pd) was added to 10 ml of DM water (based on pore volume of support: 0.22 ml/g) and also added 3 drops of 37% HCl solution. The impregnated catalyst material was allowed to dry at room temperature for 16 h and dried at 120 °C for 16 h in air-oven. The dried material was fine powdered and calcined in muffle furnace at 500 °C for 2 h with heating rate 2 °C/min in air flow (100 ml/min).

The final calculated composition of the catalyst corresponds to Pd - 0.5 wt. %, Fe₂O₃ - 1.9 wt. %, Cr - 0.65 wt. %, SO₄²⁻ - 10 wt. % with remaining part comprising ZrO₂.

### Catalyst 1-D: Pd/SO₄²⁻/Fe₂O₃-Cr₂O₃-ZrO₂

Iron and chromium promoted zirconium hydroxide (Fe-Cr-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 1.9127 g of chromium nitrate tetrahydrate Cr(NO₃)₃.9H₂O was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-chromium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min).

Palladium supported catalyst was prepared via incipient wetness impregnation method. To 44.775 g of calcined support added dropwise metal solution. The Pd solution was prepared according to this procedure: 0.3749 g PdCl₂ (0.5 wt. % Pd) was added to 10 ml of DM water (based on pore volume of support: 0.22 ml/g) and also added 3 drops of 37% HCl solution. The impregnated catalyst material was allowed to dry at room temperature for 16 h and dried at 120 °C for 16 h in air-oven. The dried material was fine powdered and calcined in muffle furnace at 550 °C for 2 h with heating rate 2 °C/min in air flow (100 ml/min).

The final calculated composition of the catalyst corresponds to Pd - 0.5 wt. %, Fe₂O₃ - 1.9 wt. %, Cr - 0.65 wt. %, SO₄²⁻ - 10 wt. % with remaining part comprising ZrO₂.

### Catalyst 1-E: Pd/SO₄²⁻/Fe₂O₃-Cr₂O₃-ZrO₂

Iron and chromium promoted zirconium hydroxide (Fe-Cr-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 1.9127 g of chromium nitrate tetrahydrate Cr(NO₃)₃.9H₂O was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-chromium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min).

Palladium supported catalyst was prepared via incipient wetness impregnation method. To 44.775 g of calcined support added dropwise metal solution. The Pd solution was prepared according to this procedure: 0.3749 g PdCl₂ (0.5 wt. % Pd) was added to 10 ml of DM water (based on pore volume of support: 0.22 ml/g) and also added 3 drops of 37% HCl solution. The impregnated catalyst material was allowed to dry at room temperature for 16 h and dried at 120 °C for 16 h in air-oven. The dried material was fine powdered and calcined in muffle furnace at 600 °C for 2 h with heating rate 2 °C/min in air flow (100 ml/min).

The final calculated composition of the catalyst corresponds to Pd - 0.5 wt. %, Fe₂O₃ - 1.9 wt. %, Cr - 0.65 wt. %, SO₄²⁻ - 10 wt. % with remaining part comprising ZrO₂.

### Catalyst 2-A: Pd/SO₄²⁻/Fe₂O₃-V₂O₅-ZrO₂

Iron and vanadium promoted zirconium hydroxide (Fe-V-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 0.9414 g of vanadyl oxalate dihydrate (VOC₂O₄.2H₂O) was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-vanadium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min).

Palladium supported catalyst was prepared via incipient wetness impregnation method. To 44.775 g of calcined support added dropwise metal solution. The Pd solution was prepared according to this procedure: 0.3749 g PdCl₂ (0.5 wt. % Pd) was added to 10 ml of DM water (based on pore volume of support: 0.22 ml/g) and also added 3 drops of 37% HCl solution. The impregnated catalyst material was allowed to dry at room temperature for 16 h and dried at 120 °C for 16 h in air-oven. The dried material was fine powdered and calcined in muffle furnace at 400 °C for 2 h with heating rate 2 °C/min in air flow (100 ml/min).

The final calculated composition of the catalyst corresponds to Pd - 0.5 wt. %, Fe₂O₃ - 1.9 wt. %, V₂O₅ - 0.75 wt. %, SO₄²⁻ - 10 wt. % with remaining part comprising ZrO₂.

### Catalyst 2-B: Pd/SO₄²⁻/Fe₂O₃-V₂O₅-ZrO₂

Iron and vanadium promoted zirconium hydroxide (Fe-V-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 0.9414 g of vanadyl oxalate dihydrate (VOC₂O₄.2H₂O) was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-vanadium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min).

Palladium supported catalyst was prepared via incipient wetness impregnation method. To 44.775 g of calcined support added dropwise metal solution. The Pd solution was prepared according to this procedure: 0.3749 g PdCl₂ (0.5 wt. % Pd) was added to 10 ml of DM water (based on pore volume of support: 0.22 ml/g) and also added 3 drops of 37% HCl solution. The impregnated catalyst material was allowed to dry at room temperature for 16 h and dried at 120 °C for 16 h in air-oven. The dried material was fine powdered and calcined in muffle furnace at 500 °C for 2 h with heating rate 2 °C/min in air flow (100 ml/min).

The final calculated composition of the catalyst corresponds to Pd - 0.5 wt. %, Fe₂O₃ - 1.9 wt. %, V₂O₅ - 0.75 wt. %, SO₄²⁻ - 10 wt. % with remaining part comprising ZrO₂.

### Catalyst 3-A: Pt/SO₄²⁻/Fe₂O₃-Cr₂O₃-ZrO₂

Iron and chromium promoted zirconium hydroxide (Fe-Cr-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 1.9127 g of chromium nitrate tetrahydrate (Cr(NO₃)₃.9H₂O) was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-chromium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min).

Platinum supported catalyst was prepared via incipient wetness impregnation method. To 44.775 g of calcined support added dropwise metal solution. The Pt solution was prepared according to this procedure: 0.6636 g of H₂PtCl₆ (0.5 wt. % Pt) was dissolved in 10 ml of DM water (based on pore volume of support: 0.22 ml/g). The impregnated catalyst material was allowed to dry at room temperature for 16 h and dried at 120 °C for 16 h in air-oven. The dried material was fine powdered and calcined in muffle furnace at 400 °C for 2 h with heating rate 2 °C/min in air flow (100 ml/min).

The final calculated composition of the catalyst corresponds to Pt - 0.5 wt. %, Fe₂O₃ - 1.9 wt. %, Cr - 0.65 wt. %, SO₄²⁻ - 10 wt. % with remaining part comprising ZrO₂.

### Catalyst 3-B: Pt/SO₄²⁻/Fe₂O₃-Cr₂O₃-ZrO₂

Iron and chromium promoted zirconium hydroxide (Fe-Cr-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 1.9127 g of chromium nitrate tetrahydrate (Cr(NO₃)₃.9H₂O) was dissolved in 975 ml of DM water. To above solution, added dropwise (30-40 min) 15 wt.% ammonium hydroxide solution (NH₄OH) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-chromium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min).

Platinum supported catalyst was prepared via incipient wetness impregnation method. To 44.775 g of calcined support added dropwise metal solution. The Pt solution was prepared according to this procedure: 0.6636 g of H₂PtCl₆ (0.5 wt. % Pt) was dissolved in 10 ml of DM water (based on pore volume of support: 0.22 ml/g). The impregnated catalyst material was allowed to dry at room temperature for 16 h and dried at 120 °C for 16 h in air-oven. The dried material was fine powdered and calcined in muffle furnace at 500 °C for 2 h with heating rate 2 °C/min in air flow (100 ml/min).

The final calculated composition of the catalyst corresponds to Pt - 0.5 wt. %, Fe₂O₃ - 1.9 wt. %, Cr - 0.65 wt. %, SO₄²⁻ - 10 wt. % with remaining part comprising ZrO₂.

### Catalyst 4-A: Pt/SO₄²⁻/Fe₂O₃-V₂O₅-ZrO₂

Iron and vanadium promoted zirconium hydroxide (Fe-V-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 0.9414 g of vanadyl oxalate dihydrate (VOC₂O₄.2H₂O) was dissolved in 975 ml of DM water. To above solution, added dropwise (30-40 min) 15 vol.% ammonium solution (NH₃) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-vanadium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min).

Platinum supported catalyst was prepared via incipient wetness impregnation method. To 44.775 g of calcined support added dropwise metal solution. The Pt solution was prepared according to this procedure: 0.6636 g H₂PtCl₆ (0.5 wt. % Pt) was dissolved in 10 ml of DM water (based on pore volume of support: 0.22 ml/g). The impregnated catalyst material was allowed to dry at room temperature for 16 h and dried at 120 °C for 16 h in air-oven. The dried material was fine powdered and calcined in muffle furnace at 400 °C for 2 h with heating rate 2 °C/min in air flow (100 ml/min).

The final calculated composition of the catalyst corresponds to Pt - 0.5 wt. %, Fe₂O₃ - 1.9 wt. %, V₂O₅ -0.75 wt. %, SO₄²⁻ - 10 wt. % with remaining part comprising ZrO₂.

### Catalyst 4-B: Pt/SO₄²⁻/Fe₂O₃-V₂O₅-ZrO₂

Iron and vanadium promoted zirconium hydroxide (Fe-V-Zr(OH)ₓ, where x balances the charge of the metals) was prepared by co-precipitation method. 130 g of zirconyl chloride octahydrate (ZrOCl₂.8H₂O), 5.39 g of iron nitrate nonahydrate (Fe(NO₃)₃.9H₂O) and 0.9414 g of vanadyl oxalate dihydrate (VOC₂O₄.2H₂O) was dissolved in 975 ml of DM water. To above solution, added dropwise (30-40 min) 15 vol.% ammonium solution (NH₃) until the pH of the resulting solution was reached to 8.71 and stirring was maintained for 60 min after complete addition. The precipitate was recovered as a wet cake via vacuum filtration, washed with 500 ml of de-ionized water to remove chloride ions and the pH was decreased to 7.0. The obtained wet cake was oven dried at 120 °C for 16 h and grounded in to fine powder. For sulfation, the dried powdered iron-vanadium-zirconium hydroxide material was added to 300 ml (292.5 ml H₂O + 8.125 ml 98% H₂SO₄) of sulfuric acid (0.5 M) and stirred for 1 h at room temperature. The slurry was filtered by vacuum filtration (no washing). The obtained wet material was dried at 120 °C for 16 h in air-oven. The obtained dried material was calcined in muffle furnace at 600 °C for 4 h with heating rate 2 °C/min in air flow (100 ml/min).

Platinum supported catalyst was prepared via incipient wetness impregnation method. To 44.775 g of calcined support added dropwise metal solution. The Pt solution was prepared according to this procedure: 0.6636 g H₂PtCl₆ (0.5 wt. % Pt) was dissolved in 10 ml of DM water (based on pore volume of support: 0.22 ml/g). The impregnated catalyst material was allowed to dry at room temperature for 16 h and dried at 120 °C for 16 h in air-oven. The dried material was fine powdered and calcined in muffle furnace at 500 °C for 2 h with heating rate 2 °C/min in air flow (100 ml/min).

The final calculated composition of the catalyst corresponds to Pt - 0.5 wt. %, Fe₂O₃ - 1.9 wt. %, V₂O₅ -0.75 wt. %, SO₄²⁻ - 10 wt. % with remaining part comprising ZrO₂.

### Catalyst Testing

The n-butane isomerization activities of the catalysts were measured in a tubular fixed-bed Inconel reactor. The details of catalyst loading and reactor were as follows: catalyst weight = 5.0 g (approx.), catalyst particle size = 0.25 - 0.5 mm, reactor ID = 16 mm, reactor OD = 19 mm. N-butane (99.9 vol. %) along-with Hydrogen (99.9 vol. %) are used as the feed. Catalyst is loaded in the reactor according to pre-determined Iso-Thermal Zone along-with inert alumina balls above and below the catalyst bed to maintain temperature/ reduce flow related mal-distribution. Prior to reaction, reduction is carried out with 16% H₂ in N₂ at 180 C for 1 hr. After reduction, reactor temperature is maintained in the range of 150-250 °C under nitrogen flow. Once desired catalyst temperature is attained, and then reactor is pressurized to 25 bar under nitrogen flow. Once attain a steady reactor temperature and pressure; both hydrogen and n-butane flow is started. Once feed flow is started, reaction exotherm is monitored along-with conversion of n-butane and selectivity towards various products v/s Time for at-least minimum 3 repeatable/ reproducible injections. After 3 repeatable injections are obtained, if needed increase temperature. Reactor heating is carried out using a Furnace, with feed and product line heating being carried out using trace heaters. Reaction conditions: catalyst weight = 5 g (0.25 - 0.5 mm), WHSV = 6 h⁻¹, H₂/N-Butane = 0.15 (mol/mol), reaction temperature for all catalysts = 235 °C.

The n-butane isomerization results (conversion, selectivity to isobutane and yield to isobutane) are given in Table 1 for comparative catalysts (A-D) and catalysts of the current invention (1-4). The data indicates that catalysts prepared using combination of iron and chromium or vanadium promoted sulfated zirconia catalysts show high performance compared to comparative examples.

**Table 1: Catalyst composition, catalyst calcination temperature and activity results of Comparative Examples and Inventive Examples**

| Catalyst | Catalyst composition | Final calcination temperature (°C) | Conversion (%) | Selectivity (%) | Yield (%) |
|---|---|---|---|---|---|
| Comparative catalyst A | Pd/SO₄²⁻/Fe₂O₃-ZrO₂ | 400 | 9.5 | 89.5 | 8.5 |
| Comparative catalyst B | Pd/SO₄²⁻/Cr₂O₃-ZrO₂ | 400 | 0.2 | 100 | 0.2 |
| Comparative catalyst C | Pd/SO₄²⁻/V₂O₅-ZrO₂ | 400 | 0.5 | 81 | 0.4 |
| Comparative catalyst D | Pd/SO₄²⁻/Fe₂O₃-Mn₂O₃-ZrO₂ | 400 | 8.1 | 89.6 | 7.2 |
| Catalyst 1-A | Pd/SO₄²⁻/Fe₂O₃-Cr₂O₃-ZrO₂ | 400 | 22.3 | 87.7 | 19.6 |
| Catalyst 1-B | Pd/SO₄²⁻/Fe₂O₃-Cr₂O₃-ZrO₂ | 450 | 23.3 | 87.0 | 20.3 |
| Catalyst 1-C | Pd/SO₄²⁻/Fe₂O₃-Cr₂O₃-ZrO₂ | 500 | 30.0 | 84.9 | 25.5 |
| Catalyst 1-D | Pd/SO₄²⁻/Fe₂O₃-Cr₂O₃-ZrO₂ | 550 | 45.0 | 79.0 | 35.5 |
| Catalyst 1-E | Pd/SO₄²⁻/Fe₂O₃-Cr₂O₃-ZrO₂ | 600 | 38.1 | 82.7 | 31.5 |
| Catalyst 2-A | Pd/SO₄²⁻/Fe₂O₃-V₂O₅-ZrO₂ | 400 | 47.3 | 75.9 | 35.9 |
| Catalyst 2-B | Pd/SO₄²⁻/Fe₂O₃-V₂O₅-ZrO₂ | 500 | 45.4 | 78.0 | 35.4 |
| Catalyst 3-A | Pt/SO₄²⁻/Fe₂O₃-Cr₂O₃-ZrO₂ | 400 | 31 | 86 | 26.7 |
| Catalyst 3-B | Pt/SO₄²⁻/Fe₂O₃-Cr₂O₃-ZrO₂ | 500 | 38.1 | 82.7 | 31.5 |
| Catalyst 4-A | Pt/SO₄²⁻/Fe₂O₃-V₂O₅-ZrO₂ | 400 | 23.5 | 87.5 | 20.6 |
| Catalyst 4-B | Pt/SO₄²⁻/Fe₂O₃-V₂O₅-ZrO₂ | 500 | 31.1 | 86.3 | 26.8 |

Although embodiments of the present application and their advantages have been described in detail, it should be understood that various changes, substitutions and alterations can be made herein without departing from the spirit and scope of the embodiments as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the above disclosure, processes, machines, manufacture, compositions of matter, means, methods, or steps, presently existing or later to be developed that perform substantially the same function or achieve substantially the same result as the corresponding embodiments described herein may be utilized. Accordingly, the appended claims are intended to include within their scope such processes, machines, manufacture, compositions of matter, means, methods, or steps.

## Claims

1. A supported catalyst composition comprising:
a sulfated support comprising an oxide or hydroxide of a group IV element and/or of aluminum, a group VIII metal oxide, and a group V metal oxide or a group VI metal oxide or both; and
a noble metal supported by the sulfated support,
wherein weight ratio of the noble metal to the group VIII metal is 1:1 to 1:5.

2. The supported catalyst composition of claim 1, wherein the composition comprises 0.1 wt. % to 1 wt. % of the noble metal.

3. The supported catalyst composition of claim 1 or 2, wherein the composition comprises 0.5 wt. % to 5 wt. % of the group VIII metal.

4. The supported catalyst composition of any one of claims 1 to 3, wherein the composition comprises 0.1 wt. % to 2 wt. % of the group V metal and/or 0.1 wt. % to 2 wt. % of the group VI metal.

5. The supported catalyst composition of any one of claims 1 to 4, wherein the composition comprises 50 wt. % to 80 wt. % of the group IV element.

6. The supported catalyst composition of any one of claims 1 to 5, wherein the composition comprises 1 wt. % to 5 wt. % of sulfur, present as a sulfate.

7. The supported catalyst composition of any one of claims 1 to 6, wherein weight ratio of the noble metal to the group VI metal is 1:0.5 to 1:1.5.

8. The supported catalyst composition of any one of claims 1 to 7, wherein weight ratio of the noble metal to the group V metal is 1:0.1 to 1:1.2.

9. The supported catalyst composition of any one of claims 1 to 8, wherein the group IV element is zirconium, preferably in the form of zirconia (ZrO₂).

10. The supported catalyst composition of any one of claims 1 to 9, wherein the noble metal is palladium (Pd) or platinum (Pt), the group VIII metal is iron (Fe), the group V metal is vanadium (V) and/or the group VI metal is chromium (Cr).

11. The supported catalyst composition of any one of claims 1 to 10, comprising:
about 0.5 wt. % Pt and a sulfated zirconia support comprising about 1.5 wt. % Fe, present as an oxide and 0.5 wt. % Cr or V, present as an oxide, wherein the weight percentages are based on total weight of the catalyst composition.

12. The supported catalyst composition of any one of claims 1 to 10, comprising:
about 0.5 wt. % Pd and a sulfated zirconia support comprising about 1.5 wt. % Fe, present as an oxide and 0.5 wt. % Cr or V, present as an oxide, wherein the weight percentages are based on total weight of the catalyst composition.

13. A process for isomerizing a C4-C7 n-paraffin hydrocarbon, the process comprising:
contacting a reactant stream comprising a C4-C7 n-paraffin hydrocarbon with the supported catalyst composition of any one of claims 1 to 12 in presence of hydrogen (H₂) under conditions suitable to isomerize at least a portion of the C4-C7 n-paraffin hydrocarbon and produce a products stream comprising a C4-C7 iso-paraffin hydrocarbon..

14. The process of claim 13, wherein the contacting condition comprises a temperature between 100 °C to 350 °C, preferably between 150 °C to 250 °C, a pressure between 100 kPa to 50 MPa, preferably between 10 MPa to 30 MPa, LHSV between 0.25 h⁻¹ to 30 h⁻¹, preferably 5 h⁻¹ to 20 h⁻¹, hydrogen to C4-C7 n-paraffin hydrocarbons molar ratio between 0.05:1 to 5:1, preferably between 0.1:1 to 1:1, or a combination thereof.

15. A process for preparing a supported catalyst composition, the process comprising:
preparing an aqueous solution comprising a group IV element salt and/or aluminum salt, a group VIII metal salt, and a group V metal salt or a group VI metal salt or both;
collecting a precipitate from the aqueous solution, wherein the precipitate comprises hydroxide(s) of the group IV element and/or of aluminum, hydroxide(s) of the group VIII metal, and hydroxide(s) of the group V metal or the group VI metal or both;
sulfating the precipitate;
drying and calcining the sulfated precipitate to obtain a sulfated support; and
adding a noble metal to the sulfated support to obtain the supported catalyst composition, wherein adding the noble metal to the sulfated support optionally comprises (i) adding a solution comprising a noble metal salt to the sulfated support to obtain a metal loaded support, (ii) drying the metal loaded support to obtain a dried metal loaded support, and (iii) calcining the dried metal loaded support to obtain the supported catalyst composition of any one of claims 1 to 12.
